(19) Europäisches Patentamt
European Patent Office
Office européen des brevets

(11) EP 1 897 915 A1

(12) EUROPEAN PATENT APPLICATION
published in accordance with Art. 153(4) EPC

(43) Date of publication:
12.03.2008 Bulletin 2008/11

(21) Application number: 06746658.1

(22) Date of filing: 19.05.2006

(51) Int Cl.:
*C09B 69/00* (2006.01)

(86) International application number:
PCT/JP2006/310051

(87) International publication number:
WO 2006/123786 (23.11.2006 Gazette 2006/47)

(84) Designated Contracting States:
DE GB

(30) Priority: 20.05.2005 JP 2005147544

(71) Applicant: Mitsubishi Kagaku Media Co., Ltd.
Tokyo
108-0014 (JP)

(72) Inventors:
- AIZAWA, Y.
KK Hayashibara Seibutsu KagakuKenkyujo
Okayama-shi,
Okayama 70000907 (JP)
- ITO, M.
KK Hayashibara Seibutsu KagakuKenkyujo
Okayama-shi,
Okayama 70000907 (JP)
- DAN-OH, Y.
KK Hayashibara Seibutsu KagakuKenkyujo
Okayama-shi,
Okayama 70000907 (JP)
- YANO, K.
KK Hayashibara Seibutsu KagakuKenkyujo
Okayama-shi,
Okayama 70000907 (JP)
- SHODA, Hisashi
c/o Mitsubishi Kagaku Media Co. Ltd
Tokyo 1080014 (JP)
- SATAKE, K.
c/o Mitsubishi Kagaku Media Co. Ltd.
Tokyo 1080014 (JP)
- UCHIDA, N.
c/o Mitsubishi Kagaku Media Co. Ltd.
Tokyo 1080014 (JP)

(74) Representative: Daniels, Jeffrey Nicholas et al
Page White & Farrer
Bedford House
John Street
London, WC1N 2BF (GB)

# (54) CYANINE COLORING MATTER AND OPTICAL RECORDING MEDIUM

(57) The present invention has an object to widen the range of applicable field of organic compounds to be selected as light absorbing materials in various fields including information recordings, solar energy generations, electric machinery apparatuses, electric communicating apparatuses, optical apparatuses, clothes, building/bedding/decorating products, sanitary and health goods, and agricultural materials, particularly, in the field of optical recording media by providing novel organic materials which absorb short-wavelength visible light, have improved lightfastness and solubility in solvents, and also have heat characteristics depending upon uses to be applied. The object is attained by providing cyanine dyes having a specific structure.

EP 1 897 915 A1

## Description

### Technical Field

The present invention relates to a novel cyanine dye and uses thereof, more particularly, to a monomethine cyanine dye which absorbs short-wavelength visible light and uses thereof.

### Background Art

In this forthcoming information age, there has been rapidly increasing in demand for organic compounds which absorb short-wavelength visible light. Now, their uses have been extended from the use such as in filter materials, where the characteristics of organic compounds that absorb and shield the visible light are utilized, to the use in information recordings, solar energy generations, etc. , where the energy of the visible light is actively used through organic compounds.

The properties that should be possessed by organic compounds being applicable to the above uses are as follows; a satisfactory light absorption property and lightfastness in short-wavelength visible region, improved solubility in solvents, and exertion of suitable heat characteristics depending on use. Representative organic compounds that have been proposed so far include, for example, anthraquinone-, phthalocyanine-, and cyanine-dyes (see, for example, Japanese Patent Kokai Nos. 116611/89, 2002-20592, and 2003-167343): Among which, anthraquinone dyes are recognized to be undesirable in light absorption property, and phthalocyanine dyes are deemed to be unfavorable in both light absorption property and solubility in solvents. While cyanine dyes have been deemed to have relatively favorable characteristics of both in light absorption property and solubility, but have troublesome in lightfastness and heat characteristics.

Among the above mentioned uses, in the field of optical recording media, particularly, in optical recording media for high-density recording that record and read information with blu-ray semiconductor laser beams, there have been expected organic compounds having the above identified properties in view of the following status.

Recently, to facilitate the realization of high-density optical recording media, there has been being developed media which can record and read information with laser beams, that emit short-wavelength light, such as blu-ray semiconductor lasers.

Organic dyes used in organic dye optical recording media, that can record and read information by using blu-ray semiconductor lasers, have maximum absorptions in the regions with shorter wavelengths than the wavelengths used for recording and reading information in conventional CD-Rs and DVD-Rs. Since such compounds have maximum absorptions in the ultraviolet region, they should inevitably have light stability against such ultraviolet rays, etc. Dyes, which have maximum absorptions in the ultraviolet region, generally have smaller molecular weights compared with dyes having maximum absorptions in the visible region, and have a restriction in their molecular designing as a problem. Hitherto, dyes such as cyanine, styryl, azo dyes, etc., have been shown that they can be used for recording and reading information by forming pits with deformation even if they have maximum absorptions in the regions with wavelengths longer than those used for recording and reading information as disclosed in, for example, Japanese Patent Kokai No. 2002-74740. Such deformation recording, however, is not favorable in terms of the quality of recording signals, and therefore a recording system without accompanying a larger deformation has been desired.

While, there have been proposed, so called, recording media with "LOW-TO-HIGH" recording mechanism, where the light absorption of dyes is decreased by laser heating to increase a light amount that reaches reflection membranes, resulting in an increment of reflection rate in recording parts. Such recording media do not require a larger absorption in the ultraviolet region and this would broaden the range of molecular designing of dyes. In optical recording media which record information by using dyes, the deterioration degree of dyes affected by blue lasers used for reading is distinctly high compared with that using conventional red-lasers (780 nm, 650 nm). In conventional methods where optical stabilizers free of affecting recording properties are added, a relatively large amount of such optical stabilizers has been required to attain a desired tolerance against light used in playback (may be designated as "playback light tolerance", hereinafter). While it has been desired to record information with an afforded lower power in terms of the maximum power of a laser used or to record information at a relatively higher speed. To meet the above, dyes feasible to react with blue-laser beams or dyes with high recording sensitivity are desirable, however, it has been deemed difficult to fulfill both such high record sensitivity and desired playback light tolerance. Particularly, in optical recording media with "LOW-TO-HIGH" recording mechanism, it may cause a problem of decreasing optical absorbance of dyes in unrecorded regions as they are repeatedly subjected to playback, resulting in an increment of reflection percentages and a reduction of signal amplitudes.

As described above, there has been desired any proposal of organic materials for recording information that overcome the above problems, have both high recording sensitivity and satisfactory playback light tolerance by using blue lasers, and have sufficient durability in actual use.

A preferable high recording sensitivity is 10 mW or lower as an index, determined on recording (1x writing speed) according to the HD DVD-R standard by using a laser beam with a wavelength of 405 nm at a numerical aperture (NA) of 0.65 and a line velocity of 6.61 m/s. This means that such high recording sensitivity can record information at 2x writing speed with a recording power of not greater than 14 mW that can be generated by general blue-lasers, considering that, when recorded at 2x writing speed (13.22 m/s), the sensitivity results in root 2 of that recorded at 1x writing speed. Recording sensitivity higher than the above level is more preferable, i.e., 9 mW or lower at 1x writing speed, more preferably, 8 mW or lower, and further preferably, 12 mW or lower at 2x writing speed. In general, in the case of constructing a recording layer in a bilayer or multilayer form, as the number of layers increases, the more the number of layers the lower the light amount, i.e., energy required for recording information, used for recording information in each layer becomes due to light absorption and reflection by layers other than a layer for recording, as well as a reduction of light amount induced by light transmittance from the recording layer to a deeper layer(s). Thus, it is preferable that dyes *per se* should have a higher recording sensitivity.

Disclosure of Invention

In view of these circumstances, an object of the present invention is to widen the applicable range of organic compounds selectable as light-absorbing materials in the above described fields, particularly, in optical recording media by providing novel organic compounds which absorb short-wavelength visible light, have satisfactory lightfastness and solubility in solvents, and have heat characteristics depending upon application to which the organic compounds are applied.

The present inventors eagerly researched and screened cyanine dyes which had been regarded as being poor in lightfastness and heat characteristics, leading to the finding that specific monomethine cyanine dyes, which have metal complexes as anions, indolenine rings at both ends of their monomethine chains, and substituents with cyclic structures that bind to the C-3 positioning at least either of their indolenine rings, have satisfactory lightfastness, efficiently absorb the visible light ranging from the ultraviolet to the blue regions, exhibit satisfactory solubility in organic solvents with no problem in actual use, and have satisfactory heat characteristics. Thus, it was revealed that such cyanine dyes can be advantageously used as novel light-absorbing materials, which absorb short-wavelength visible light for shielding it or utilizing its energy, in a variety of fields that require organic compounds with these properties.

The present inventors found that the use of the above-identified organic compounds according to the present invention provides optical recording media which have both satisfactory playback light tolerance and sensitivity at high speed recording, and thus they accomplished this invention. As described above, the following three items can be listed as required properties requisite for blu-ray semiconductor laser recording materials; satisfactory recording properties, sufficient playback light tolerance, and recording sensitivity suitable for high speed recording. In the present invention, it was found that the above three required properties are satisfied at the same time by allocating the desired functions to both cationic and anionic components in an appropriate combination. It was conducted a material designing, where the contribution of absorption in a thin layer form at a laser beam wavelength of 405 nm was more lowered than that of a cationic part and the function of playback light tolerance was more weighed than that of recording properties, in such a manner of selecting a material, as a cyanine compound for a cationic component, which has optical properties suitable for "LOW-TO-HIGH" recording mechanism and has an absorption maximum at around the wavelength for reading a recorded information when in a thin layer form; and selecting a material, as a metal complex for an anionic component, which has a maximum absorption at a longer wavelength than that of the cationic component, whereby both satisfactory recording properties and sufficient playback light tolerance were attained. Considering an appropriate combination of the cationic and anionic parts, dyes with high sensitivity were obtained by adjusting their heat decomposition points to around 200 to 350°C in order to obtain recording sensitivity suitable for high speed recording.

The present invention solves the above object by providing the cyanine dyes represented by General Formula 1.

The present invention also solves the above object by providing optical recording media containing cyanine dyes represented by General Formula 1.

General Formula 1:

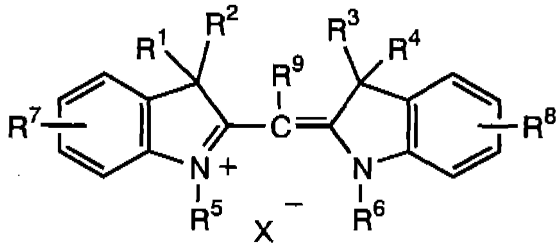

(wherein in General Formula 1, $R^1$ through $R^4$ are the same or different hydrocarbon groups, and at least one of which either binds to a cyclic group that may optionally have a substituent or form a cyclic structure by binding together with other hydrocarbon group that binds to the same carbon atom. $R^5$ and $R^6$ are the same or different hydrocarbon groups that may have substituents. $R^7$ through $R^9$ represent hydrogen atoms or appropriate substituents. $X^-$ represents an anion of metal complex having, as a central atom, a transition element from the 5 to 12 groups in the periodic law table.)

Brief Description of Drawing

FIG. 1 is a figure which shows the recording power of a laser beam and PRSNR.

Best Mode for Carrying Out the Invention

As mentioned above, the present invention relates to the cyanine dyes represented by General Formula 1.

General Formula 1:

[Chem. 2]

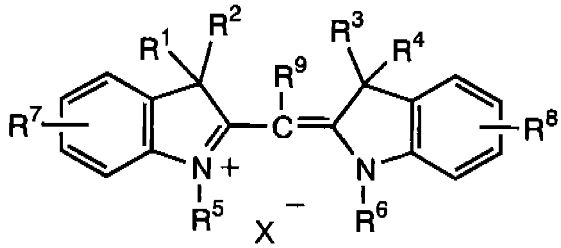

wherein in General Formula 1, $R^1$ through $R^4$ represent the same or different hydrocarbon groups. Examples of such hydrocarbon groups of $R^1$ through $R^4$ are those which have straight or branched hydrocarbon groups having six or lower carbon atoms; aliphatic hydrocarbon groups such as methyl, ethyl, propyl, isopropyl, isopropenyl, 1-propenyl, 2-propenyl, 2-propynyl, butyl, isobutyl, *sec*-butyl, *tert*-butyl, 2-butenyl, 1,3-butadienyl, pentyl, isopentyl, neopentyl, *tert*-pentyl, 1-methyl pentyl, 2-methyl pentyl, and 2-pentene-4-inyl groups; and alicyclic hydrocarbon groups such as cyclopropyl, cyclobutyl, cyclopenthyl, cyclohexyl, and cyclohexenyl groups. Among $R^1$ through $R^4$, at least one of which either binds to a saturated or unsaturated cyclic group such as azulene, quinoline, cyclopropane, cyclobutane, cyclopentane, cyclohexane, cyclohexene, naphthalene, thiophene, benzene, piperidine, pyridine, pyrolidine, pyrrole, and furan rings; or binds together with other hydrocarbon group that binds to the C-3 in the same indolenine ring to form, for example, a saturated or unsaturated cyclic structure such as cyclobutane, cyclopentane, cyclohexane, cyclohexene, and cycloheptane rings. In the case that the hydrocarbon groups of $R^1$ through $R^4$ have cyclic groups, such cyclic groups may have one or more substituents as long as they do not affect the object of the present invention. Examples of such substituents are aliphatic hydrocarbon groups such as methyl, ethyl, propyl, isopropyl, isopropenyl, 1-propenyl, 2-propenyl, 2-propynyl, butyl, isobutyl, *sec*-butyl, *tert*-butyl, 2-butenyl, 1,3-butadienyl, pentyl, isopentyl, neopentyl, *tert*-pentyl, 1-methyl pentyl,

2-methyl pentyl, and 2-pentene-4-inyl groups; alicyclic hydrocarbon groups such as cyclopropyl, cyclobutyl, cyclopenthyl, cyclohexyl, and cyclohexenyl groups; aromatic hydrocarbon groups such as phenyl, *o*-tolyl, *m*-tolyl, *p*-tolyl, xylyl, mesityl, *o*-cumenyl, *m*-cumenyl, *p*-cumenyl, and biphenylyl groups; halogen groups such as fluoro, chloro, bromo, and iodo groups; and combinations thereof.

$R^5$ and $R^6$ in General Formula 1 represent the same or different hydrocarbon groups which may have one or more substituents. Examples of such hydrocarbon groups are those which have straight or branched hydrocarbon groups having six or lower carbon atoms; aliphatic hydrocarbon groups such as methyl, ethyl, propyl, isopropyl, isopropenyl, 1-propenyl, 2-propenyl, 2-propynyl, butyl, isobutyl, *sec*-butyl, *tert*-butyl, 2-butenyl, 1,3-butadienyl, pentyl, isopentyl, neopentyl, *tert*-pentyl, 1-methyl pentyl, 2-methyl pentyl, and 2-pentene-4-inyl groups; and alicyclic hydrocarbon groups such as cyclopropyl, cyclobutyl, cyclopenthyl, cyclohexyl, and cyclohexenyl groups; aromatic hydrocarbon groups such as phenyl, o-tolyl, m-tolyl, p-tolyl, xylyl, mesityl, o-cumenyl, m-cumenyl, p-cumenyl, and biphenylyl groups; where one or more of the hydrogen atoms in the above hydrocarbon groups may be substituted with a halogen group(s) such as fluoro, chloro, and bromo groups.

$R^7$ through $R^9$ in General Formula 1 each independently represent hydrogen atoms or appropriate substituents. Examples of such substituents are aliphatic hydrocarbon groups such as methyl, ethyl, propyl, isopropyl, isopropenyl, 1-propenyl, 2-propenyl. 2-propynyl, butyl, isobutyl, sac-butyl, *tert*-butyl, 2-butenyl, 1,3-butadienyl, pentyl, isopentyl, neopentyl, *tert*-pentyl, 1-methyl pentyl, 2-methyl pentyl, and 2-pentene-4-inyl groups; and alicyclic hydrocarbon groups such as cyclopropyl, cyclobutyl, cyclopenthyl, cyclohexyl, and cyclohexenyl groups; aromatic hydrocarbon groups such as phenyl, *o*-tolyl, *m*-tolyl, *p*-tolyl, xylyl, mesityl, o-cumenyl, m-cumenyl, *p*-cumenyl, and biphenylyl groups; ether groups such as methoxy, ethoxy, propoxy, isopropoxy, butoxy, isobutoxy, *sec*-butoxy, *tert*-butoxy, pentyloxy, and phenoxy groups; ester groups such as methoxycarbonyl, ethoxycarbonyl, propoxycarbonyl, acetoxy, and benzoyloxy groups; amino groups such as dimethylamino, diethylamino, dipropylamino, diisopropylamino, dibutylamino, and dipentylamino groups; halogen groups such as fluoro, chloro, bromo, and iodo groups; hydroxy group; carboxy group; cyano group; nitro group; and combinations thereof.

$X^-$ in General Formula 1 represents an anion of metal complex having, as a central atom, a transition element from the 5 to 12 groups in the periodic law table. Examples of such metal complex are transient metal chelates such as of acetylacetonato chelate, azo, salicylaldehyde oxime, diimmonium, dithiol, dipyrromethene, squalirium, thiocatechol chelate, thiobisphenolate chelate, bisdithio-$\alpha$-diketone chelate, bis phenylene dithiol, and formazan. Among which, azo transient metal chelates are preferable in view of lightfastness and solubility in solvents of the cyanine dyes as a whole. Examples of transient elements in the above-identified metal complexes are vanadium, niobium, tantalum, chromium, molybdenum, tungsten, manganese, technetium, rhenium, iron, ruthenium, osmium, cobalt, rhodium, iridium, nickel, palladium, platinum, copper, silver, gold, zinc, cadmium, and mercury. Among which, vanadium, manganese, cobalt, and copper are preferable from economical viewpoints and influence on living bodies.

Preferable azo metal complexes are, for example, those represented by General Formulae 2 to 5:

General Formula 2:

[Chem. 3]

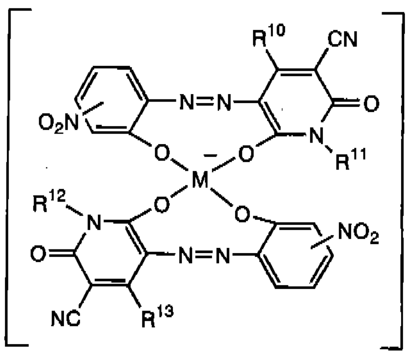

General Formula 3:

[Chem. 4]

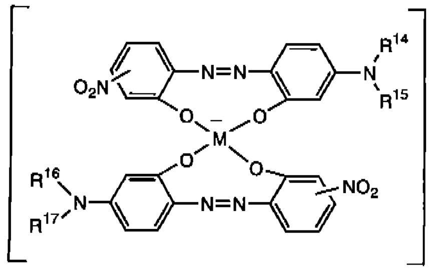

General Formula 4:

[Chem. 5]

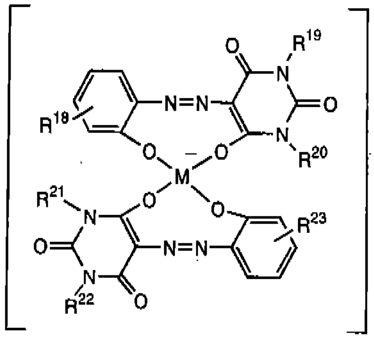

General Formula 5:

[Chem. 6]

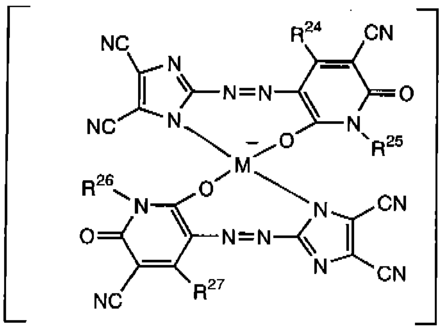

Throughout General Formulae 2 to 5, M represents any of the above-mentioned transient elements from the 5 to 12 groups in the periodic law table.

$R^{11}$, $R^{12}$, $R^{14}$ to $R^{17}$, $R^{19}$ to $R^{22}$, $R^{25}$ and $R^{26}$ in General Formulae 2 to 5 represent the same or different hydrocarbon groups within the same molecule. Respective examples of such hydrocarbons are those which have straight or branched chains having six or lower carbon atoms; aliphatic hydrocarbon groups such as methyl, ethyl, propyl, isopropyl, isopropenyl, 1-propenyl, 2-propenyl, 2-propynyl, butyl, isobutyl, *sec*-butyl, *tert*-butyl, 2-butenyl, 1,3-butadienyl, pentyl, isopentyl, neopentyl, *tert*-pentyl, 1-methyl pentyl, 2-methyl pentyl, and 2-pentene-4-inyl groups; and alicyclic hydrocarbon groups such as cyclopropyl, cyclobutyl, cyclopenthyl, cyclohexyl, and cyclohexenyl groups; wherein one or more of the hydrogen atoms in the above hydrocarbon groups may be substituted with a halogen group(s) such as fluoro, chloro, and bromo groups. Although it varies depending on the types of solvents used, the solubility in solvents of the cyanine dyes of the present invention generally increases as the number of carbon atoms in $R^{11}$, $R^{12}$, $R^{14}$ to $R^{17}$, $R^{19}$ to $R^{22}$, $R^{25}$ and $R^{26}$ increases.

$R^{10}$, $R^{13}$, $R^{18}$, $R^{23}$, $R^{24}$ and $R^{27}$ in General Formulae 2, 4 and 5 represent hydrogen atoms or the same or

different substituents within the same molecule, and respective examples of such substituents include aliphatic hydrocarbon groups such as methyl, ethyl, propyl, isopropyl, isopropenyl, 1-propenyl, 2-propenyl, 2-propynyl, butyl, isobutyl, *sec*-butyl, *tert*-butyl, 2-butenyl, 1,3-butadienyl, pentyl, isopentyl, neopentyl, *tert*-pentyl, 1-methyl pentyl, 2-methyl pentyl, and 2-pentene-4-inyl groups; and alicyclic hydrocarbon groups such as cyclopropyl, cyclobutyl, cyclopenthyl, cyclohexyl, and cyclohexenyl groups; aromatic hydrocarbon groups such as phenyl, *o*-tolyl, *m*-tolyl, *p*-tolyl, xylyl, mesityl, *o*-cumenyl, *m*-cumenyl, *p*-cumenyl, and biphenylyl groups; ether groups such as methoxy, ethoxy, propoxy, isopropoxy, butoxy, isobutoxy, *sec*-butoxy, *tert*-butoxy, pentyloxy, and phenoxy groups; ester groups such as methoxycarbonyl, ethoxycarbonyl, propoxycarbonyl, acetoxy, and benzoyloxy groups; amino groups such as dimethylamino, diethylamino, dipropylamino, diisopropylamino, dibutylamino, and dipentylamino groups; halogen groups such as fluoro, chloro, bromo, and iodo groups; hydroxy group; carboxy group; cyano group; nitro group; and combinations thereof.

In the anions of azo metal complexes represented by General Formulae 2 and 3, the positions to which two nitro groups bind should not specifically be restricted to any of ortho-, meta-, and para-positions with respect to azo group, however, the meta-position is preferable from a synthetic point of view.

Concrete examples of the cyanine dyes of the present invention are, for example, those which are represented by Chemical Formulae 1 to 30. All of these compounds have predominant absorption maxima at wavelengths of over 400 nm when in a solution form, usually, in the violet to the blue regions at wavelengths of around 400 to 500 nm, and have molecular absorption coefficients of 5 x $10^4$ or higher, usually, 7 x $10^4$ or higher at their absorption maxima, meaning that they efficiently absorb the visible right in the violet to the blue regions.

Chemical Formula 1:

[Chem. 7]

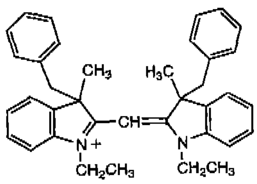
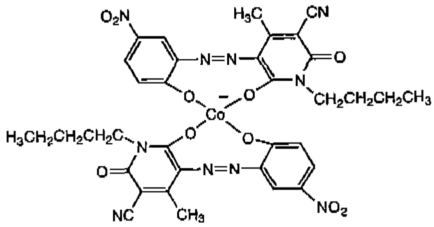

Chemical Formula 2:

[Chem. 8]

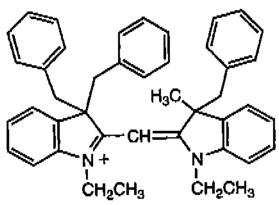
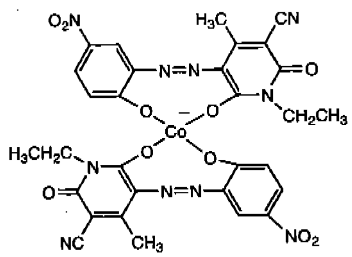

Chemical Formula 3:

[Chem. 9]

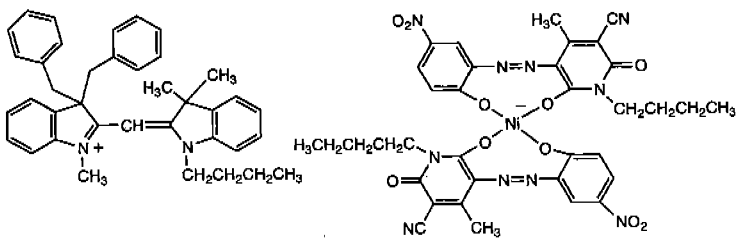

Chemical Formula 4:

[Chem. 10]

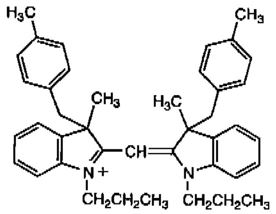

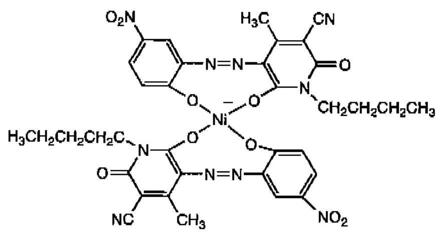

Chemical Formula 5:

[Chem. 11]

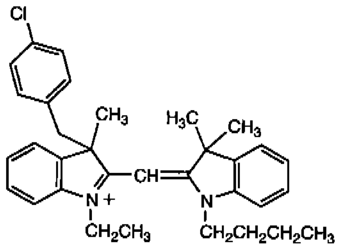

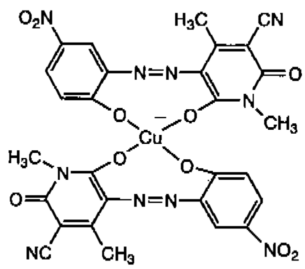

Chemical Formula 6:

[Chem. 12]

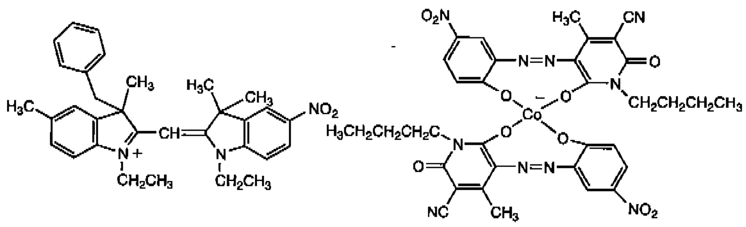

Chemical Formula 7:

[Chem. 13]

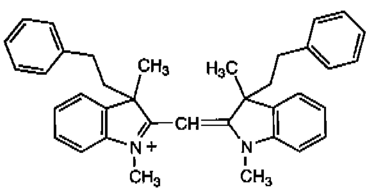

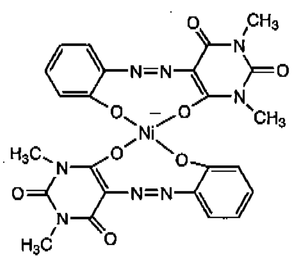

Chemical Formula 8:
[Chem. 14]

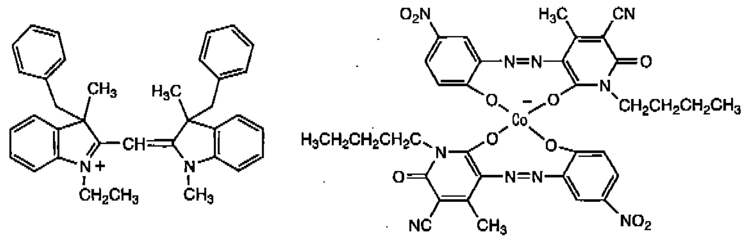

Chemical Formula 9:
[Chem. 15]

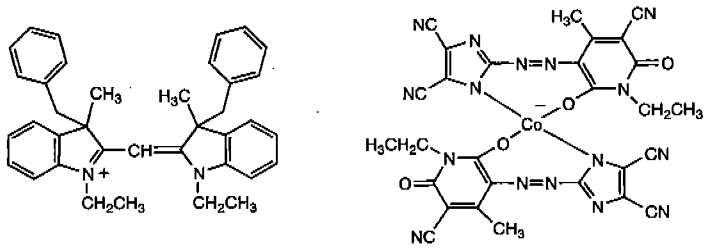

Chemical Formula 10:
[Chem. 16]

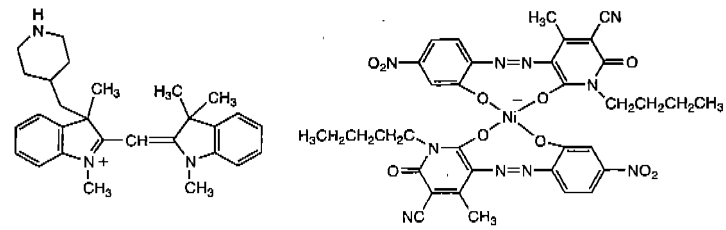

Chemical Formula 11:
[Chem. 17]

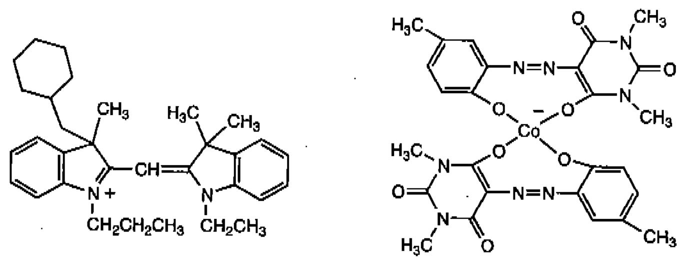

Chemical Formula 12:
[Chem. 18]

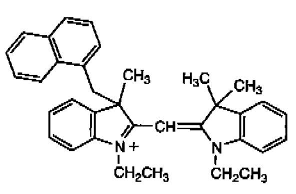

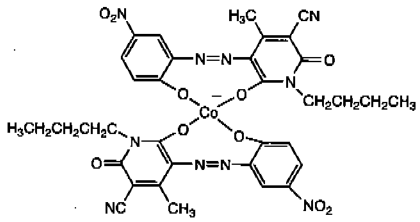

Chemical Formula 13:
[Chem. 19]

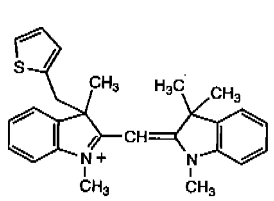

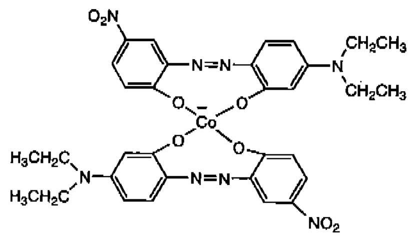

Chemical Formula 14:
[Chem. 20]

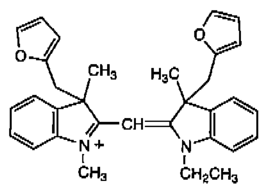

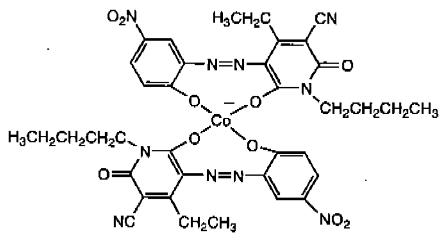

Chemical Formula 15:
[Chem. 21]

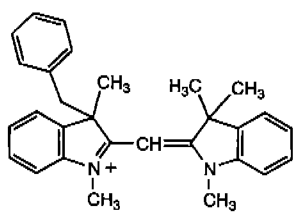

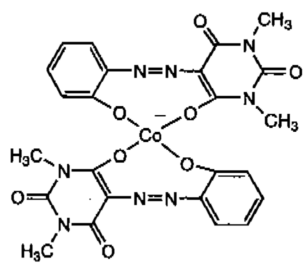

Chemical Formula 16:
[Chem. 22]

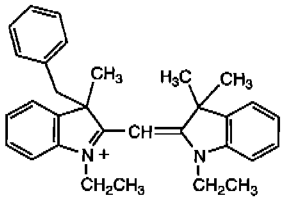

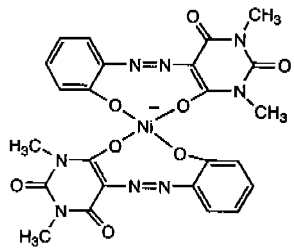

Chemical Formula 17:
[Chem. 23]

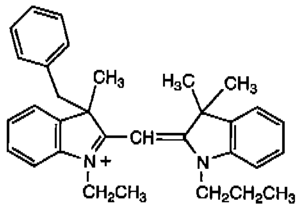

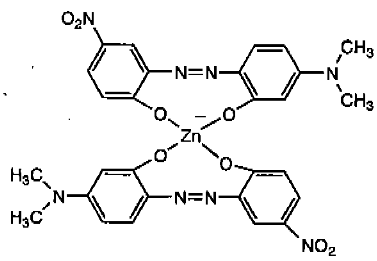

Chemical Formula 18:
[Chem. 24]

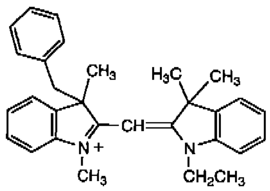

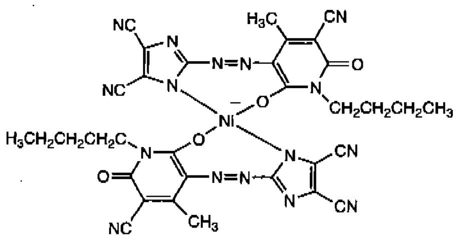

Chemical Formula 19:
[Chem. 25]

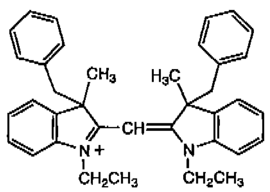

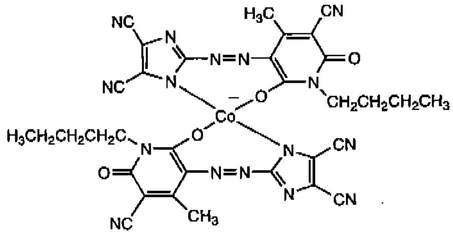

Chemical Formula 20:
[Chem. 26]

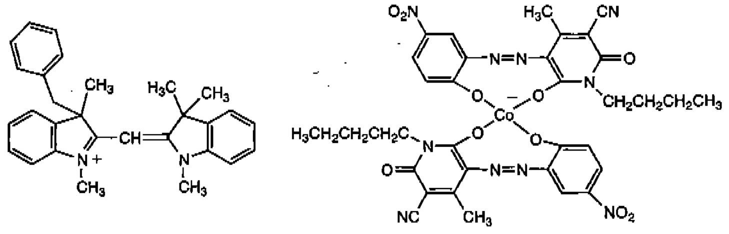

Chemical Formula 21:
[Chem. 27]

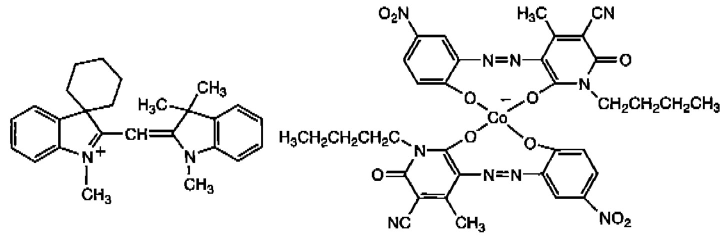

Chemical Formula 22:
[Chem. 28]

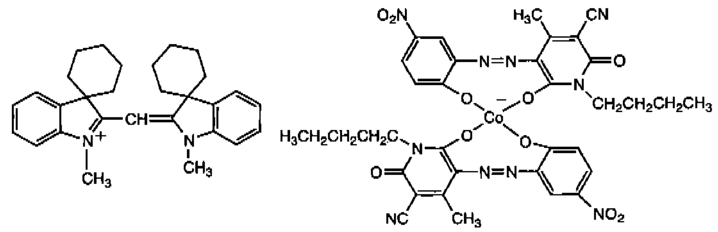

Chemical Formula 23:
[Chem. 29]

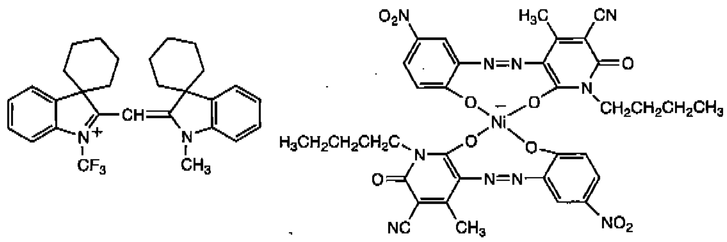

Chemical Formula 24:
[Chem. 30]

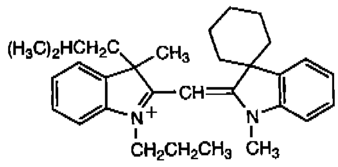

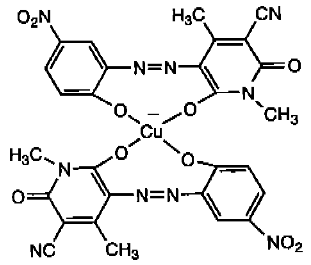

Chemical Formula 25:
[Chem. 31]

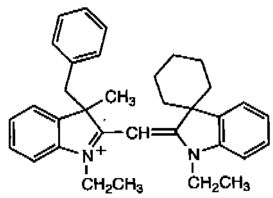

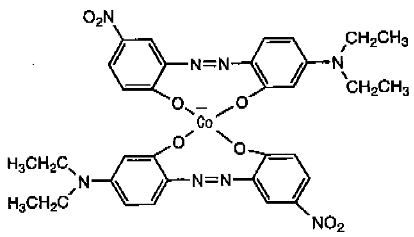

Chemical Formula 26:
[Chem. 32]

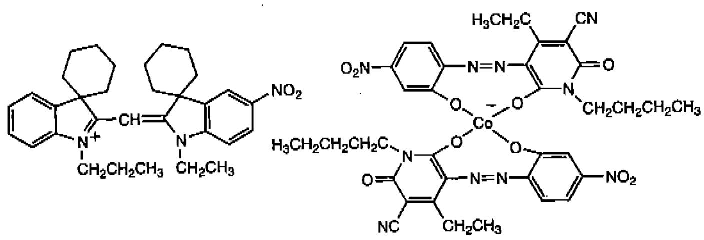

Chemical Formula 27:
[Chem. 33]

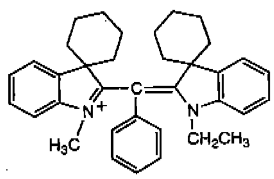

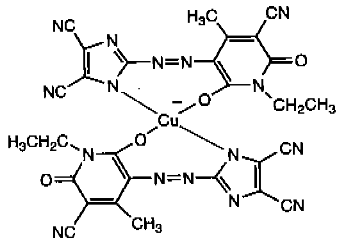

Chemical Formula 28:
[Chem. 34]

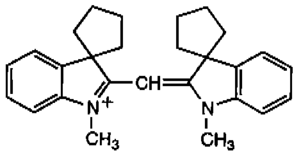

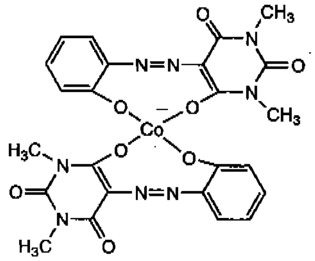

Chemical Formula 29:
[Chem. 35]

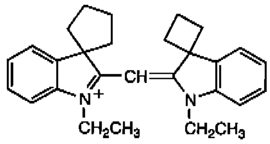

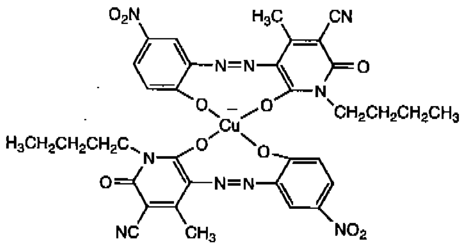

Chemical Formula 30:
[Chem. 36]

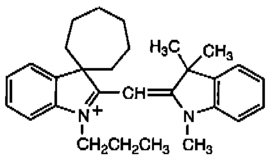

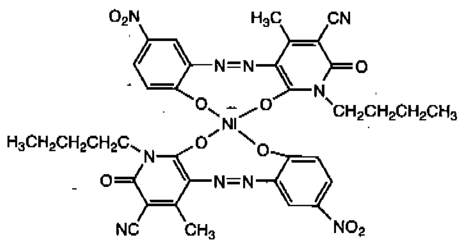

The synthetic method of the cyanine dyes of the present invention is shown below but it should never be restricted thereunto.

The cyanine dyes of the present invention can be obtained, for example, through a step of reacting the compounds, represented by General Formula 6 having $R^1$, $R^2$, $R^5$, $R^7$ and $R^9$ which correspond to those in General Formula 1, with the compounds represented by General Formula 7 having $R^3$, $R^4$, $R^6$ and $R^8$ which correspond to those in General Formula 1. $X^-$ in General Formula 7 includes appropriate anions, for example, inorganic acid ions such as fluoro, chloro, bromo, iodo, fluorine, chloric acid, bromic acid, iodic acid, perchloric acid, periodic acid, phosphoric acid, phosphoric acid hexafluoride, antimony hexafluoride, tin acid hexafluoride, fluoroboric acid, and tetrafluoroborate ions; and organic acid ions such as thiocyanic acid, benzenesulfonic acid, naphthalenesulfonic acid, naphthalenedisulfonic acid, benzenecarboxylic acid, alkylcarboxylic acid, trihaloalkylcarboxylic acid, alkylsulfonic acid, trihaloalkylsulfonic acid, nicotinic acid, and tetracyanoquinodimethane ions.

General Formula 6:

[Chem. 37]

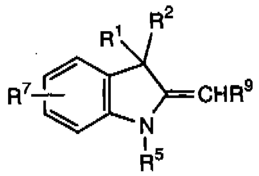

General Formula 7:

[Chem. 38]

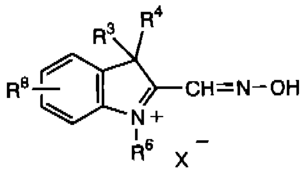

In the synthesis, the compounds represented by General Formulae 6 and 7 are placed in a reaction vessel in an appropriate amount, respectively, and optionally dissolved in an appropriate solvent, and allowed to react at ambient temperature or higher while heating and stirring in such a manner of heat refluxing.

Examples of such a solvent are hydrocarbon solvents such as pentane, hexane, cyclohexane, petroleum ether, octane, petroleum benzine, isooctane, benzene, toluene, and xylene; halides such as carbon tetrachloride, chloroform, 1,2-dichloroethane, 1,2-dibromoethane, trichloroethylene, tetrachloroethylene, chlorobenzene, bromobenzene, and $\alpha$-dichlorobenzene; alcohols and phenols such as methanol, ethanol, 2,2,2-trifluoroethanol, 1-propanol, 2-propanol, 1-butanol, 2-butanol, isobutylalcohol, isopentylalcohol, cyclohexanol, ethylene glycol, propylene glycol, 2-methoxyethanol, 2-ethoxyethanol, phenol, benzyl alcohol, cresol, diethylene glycol, triethylene glycol, and glycerin; ethers such as diethyl ether, diisopropyl ether, tetrahydrofuran, tetrahydropyran, 1,4-dioxane, anisole, 1,2-dimethoxyethane, diethylene glycol dimethyl ether, dicyclohexyl-18-crown-6, methylcarbitol, and ethylcarbitol; acids and derivatives thereof such as acetic acid, acetic anhydride, trichloroacetic acid, trifluoroacetic acid, ethyl acetate, butyl acetate, ethylene carbonate, propylene carbonate, formamide, N-methylformamide, N,N-dimethylformamide, N-methylacetamide, N,N-dimethylacetamide, hexamethylphosphoric triamide, and triethyl phosphate; nitriles such as acetonitrile, propionitrile, succinonitrile, and benzonitrile; amines such as ethylenediamine, diisopropylethylamine, triethylamine, pyridine, piperidine, and morpholine; nitro compounds such as nitromethane and nitrobenzene; ketones such acetone and methylethylketone; sulfur-containing compounds such as dimethylsulfoxide and sulfolane; and water, which all may be used in combination, if necessary.

In the case of using solvents, generally, a larger amount of solvent results in a lesser reaction efficiency, while a lesser amount of solvent results in a more difficulty in homogenous heating and stirring and also results in a more liability to side reactions. Thus, it is desirable to set the amount of a solvent to a level of up to 100-folds, usually, 5 to 50-folds by weight against the total amounts of material compounds used. The reaction completes within 10 hours, usually, within five hours, depending on the types of material compounds and reaction conditions. The progress of reaction can be monitored by conventional method, for example, thin layer chromatography, gas chromatography, and high-performance liquid chromatography. When the cyanine dyes thus obtained are in the form of any of salts with anions of organic or inorganic acids other than the specific anions of metal complexes, they are allowed to react with onium salts of the above metal complexes such as those of ammonium salt, pyridinium salt, and quinolinium salt to exchange the organic or inorganic anions with the desired anions of metal complexes. The compounds represented by General Formulae 6 and 7 can be obtained in accordance with the method disclosed in, for example, "KANKO-SHIKISO (Photosynthesizing Dyes)", pp. 24-30, published by Sangyo Tosho Publisher, Tokyo, Japan, edited by Masaaki HAYAMI, October 17, 1997. In the case that commercialized products thereof are available, they can be used after purification, if necessary.

The cyanine dyes thus obtained can be used intact in a reaction mixture form depending on use, and prior to use, they are usually subjected to conventional methods for purifying related compounds, such as dissolution, separation, decantation, filtration, extraction, concentration, thin layer chromatography, column chromatography, gas chromatography, high-performance liquid chromatography, distillation, sublimation, and crystallization which may be applied in combination, if necessary. Depending on the type of the cyanine dyes and uses thereof, when they are applied to information

recordings and solar energy generations that require high purity organic dye compounds, they are desirably purified with, for example, distillation, sublimation, and crystallization, prior to use.

As described above, the cyanine dyes according to the present invention effectively absorb the following visible light because they have predominant absorption maxima at wavelengths of longer than 400 nm, usually, at wavelengths of around 400 to 500 nm in the violet to the blue regions, and have distinctly high molecular absorption coefficients at wavelengths of their absorption maxima (may be designated as "ε", hereinafter) of least $5 \times 10^4$, usually, at least $7 \times 10^4$. Also, the cyanine dyes of the present invention have satisfactory solubility with no actual hindrance when dissolved in organic solvents such as those of amides, alcohols, ketones, nitriles, and halogens, which are often used in the fields of, for example, information recordings and solar energy generations; and most of which have decomposition points of 250°C or higher. As well known, the decomposition point of organic compounds is recognized as an important index of heat characteristics of organic compounds, and it is said that organic compounds with higher decomposition points have higher thermal stability. Thus, the cyanine dyes of the present invention are distinctly useful as absorption materials which absorb short-wavelength visible light for shielding it and use the energy of such visible light in various fields including information recordings, solar energy generations, electric machinery apparatuses, electric communicating apparatuses, optical apparatuses, clothes, building/bedding/decorating products, sanitary and health goods, and agricultural materials. The decomposition points of organic dye compounds such as cyanine dyes can be determined on, for example, commonly used differential scanning calorimetric analysis (abbreviated as "DSC", hereinafter).

The cyanine dyes of the present invention are useful in the field of information recording as photosensitizers or heat exchange agents for promoting polymerization by absorbing short-wavelength visible light and sensitizing polymerizable compounds and polymerization initiators which are used in optical cards, plate makings, thermal transfer recordings, thermal recordings, and hologram recordings. As for other uses as photosensitizers, for example, in the field of solar energy generations, the cyanine dyes of the present invention increase the sensitivity of semiconductor electrodes against short-wavelength visible light and distinctly improve the photoelectric conversion efficiency of solar cells, when allowed to be supported on semiconductor electrodes of dye-sensitive wet-type solar cells. Since the cyanine dyes of the present invention exert satisfactory lightfastness with no actual hindrance against environmental light such as natural- and artificial-light, solar cells with the cyanine dyes as photosensitizers have the advantage that they hardly cause reduction in electromotive force inducible by the photosensitizers even when used for a relatively long period of time.

In the fields of electric communicating apparatuses, electric machinery apparatuses, and optical apparatuses, the cyanine dyes of the present invention have the merits that they can decrease both the noises due to the visible light and the elevation of ambient temperature due to a radiated heat ray, and control the visibility to a desired level when applied to, for example, pickup tubes, semiconductor light-receiving elements, and optical fibers as filter materials. As to another use of the cyanine dyes as filter materials, in the field of agricultural materials, the cyanine dyes can control the growth of the following plants by regulating the wavelength distribution of light to be irradiated on useful plants such as decorative-, gardening-, edible-, and medicinal-plants including orchards, crops, vegetables, and flowers by coating onto, for example, a glass plate for greenhouses and plastic base-materials for plastic greenhouses formed in a sheet or film form.

In addition to the above uses, the cyanine dyes according to the present invention have the merits that, when optionally used in combination with one or more other materials which absorb the light in the regions of ultraviolet rays, visible light and/or infrared rays, they can prevent or reduce troubles and discomforts such as undesirable temperature changing and visible-light-inducible eye strain/senescence of optic nerve/cataract in organisms and products; modify the chromaticity, color tone, tint, and appearance; and maintain or control the reflected or transmitted light through the products to a desired level when used as a light-shading agent, heat-ray shading agent, thermal insulator, and heat reserving and storing agent in clothes in general, particularly, clothes using heat reserving and storing fiber or using fibers having a disguising property against a reconnaissance using ultraviolet rays, visible light, and infrared rays; and products other than clothes including, for example, building/bedding/decorating products such as a casement, shirring, drape, pleat, print, venetian blind, lace, Roman Shade, roller blind, shutter, store curtain, blanket, futon, peripheral material for thick bed quilt, quilt cover, bed sheet, cushion, pillow, pillow cover, decorative pillow, mat, carpet, sleeping bag, windowpane, interior- and exterior-finish for buildings/cars/automobiles/trains/ships/airplanes, and window glass; sanitary and health goods such as a paper diaper, diaper cover, eyeglasses, monocle, and lorgnette; internal base sheets, linings, and materials for shoes; wrappers; materials for umbrellas; parasols; stuffed toys; lighting devices; sunglasses; sun visors; sunroofs; peeping windows of microwaves and ovens; and wrapping- and injection-materials and vessels for enclosing the above products. The cyanine dyes of the present invention would also be useful as an indelible ink, bar-code ink for falsification, light-absorbing ink, absorption paint, marking agent for locating pictures or films, sorting stain for recycling plastics, preheating assisting agent for fabricating plastic bottles, and active ingredient of drugs for treating tumors in general which may have thermo-sensitivity against visible light.

Although the cyanine dyes of the present invention have distinct lightfastness against environmental light such as natural- and artificial-light, when applied to the above-identified uses, the present invention should never exclude embodiments, which optionally use one or more lightfastness improvers, i.e., quenchers in combination to prevent the

fading, deterioration, change in property and quality, decomposition, etc., of the cyanine dyes, induced by singlet oxygen which may be generated by irradiation of laser beams, etc. Examples of such lightfastness improvers used in combination with the cyanine dyes of the present invention include, for example, amine compounds, carotenoid compounds, sulfide compounds, and phenolic compounds, as well as inorganic chelate complexes and organic metal complexes, such as of acetylacetonato chelate, salicylaldehyde oxime, diinmonium, dithiol, catechol chelate, thiobisphenolate chelate, bisthio-a-diketone chelate, and formazan, which are disclosed in, for example, Saikohyo Patent Publication No. WO 00/075111, applied for by the same applicant as the present applicant; "Shikizai-Kogaku-Handbook" (Handbook for Color Material Technology). 1st edition, edited by Japan Society of Color Material, pp. 1,274-1,282, published by Asakura Publishing Co., Ltd., November 25, 1989; and "Senryo-to-Yakuhin" (Dyes and Chemicals), edited by Masahiro SHINKAI et. al., Vol. 37, No. 7, pp. 185-197 (1992), and which all can be optionally used in combination. Among which, metal complexes of formazan and dithiol are particularly preferable because they distinctly improve the lightfastness of the cyanine dyes of the present invention and realize the formation of satisfactory amorphous solids when in a mixture condition with any of the cyanine dyes. Varying depending on use, the amount of any of the lightfastness improvers used in combination with the cyanine dyes of the present invention is usually increased or decreased within the range of one percent by weight or more, preferably, 3 to 30% to the amount of the cyanine dye(s). In the case of using the lightfastness improvers in combination, the cyanine dyes of the present invention are applied to the desired products by previously mixing to homogeneity with the lightfastness improvers into a liquid, semi-solid, or solid composition; or applied to the desired products separately with the lightfastness improver(s) while controlling the composition ratio of the cyanine dye(s) of the present invention and the lightfastness improver(s) by increasing or decreasing the amount of these compounds to form a liquid, semi-solid or solid composition.

The following are the optical recording media useful as applications of the cyanine dyes of the present invention.

Although the type and the recording/reading system of the optical recording media, which use the cyanine dyes of the present invention as compounds for recording layers, should never be restricted, the cyanine dyes are particularly suitable for optical recording media which record/read information using a light with a wavelength of 300 nm or longer but not longer than 500 nm. The cyanine dyes are useful for recording/reading information using a light with a wavelength of 400 nm or longer and suitable for optical recording media which record/read information using blu-ray semiconductor lasers. Concretely, they are useful for HD DVD-R. The cyanine dyes are also useful for multi-layered recording media whose recording layers are multi-layered.

As described above there can be listed the following three items of required characteristics requisite for optical recording materials for blu-ray semiconductor lasers; an improved recording property, sufficient durability against reading light, and recording sensitivity suitable for high speed recording. The present invention attained simultaneous fulfillment of the above required characteristics by combining cationic and anionic components in the cyanine dyes in such a manner of allocating the required functions to these components, respectively. In concrete, it was conducted a material designing, where the function in terms of stability against reading light is more weighed than recording property, in such a manner of selecting materials with optical properties suitable for "LOW-TO-HIGH" recording mechanism which employs, as cation components, cyanine dyes having absorption maxima at around the wavelength used for recording/reading information when in a thin layer form; and selecting materials, as anionic components, i.e., metal complexes, which have absorption maxima longer than those of the cationic components, when in a thin layer form, to lower the donation of anionic components compared with that of cationic components in terms of the absorption level at a laser beam wavelength of 405 nm, when in a thin layer form. This enabled a mutual establishment of the desired improved recording property and sufficient durability against reading light. The present inventors further studied a desired combination of the cationic and anionic components and attained the desired high sensitivity by controlling the thermal decomposition points of dyes at around 200 to 350°C in order to obtain a recording sensitivity suitable for high speed recording.

Examples of the cyanine dyes of the present invention for use in optical recording media can be used by appropriately selecting from among the above-described compounds. Among which, those represented by General Formula 1, where at least one of $R^1$ through $R^4$ is one which binds to a saturated or unsaturated cyclic group. Examples of such cyclic groups are azulene, quinoline, cyclopropane, cyclobutane, cyclopentane, cyclohexane, cyclohexene, naphthalene, thiophene, benzene, piperidine, pyridine, pyrrolidine, pyrrole, and furan rings. In particular, preferable compounds are those with aromatic rings as cyclic rings, and those with benzene ring are more preferable. Compounds, where at least one of $R^1$ through $R^4$ is benzyl group, are preferable.

Since the optical recording media, which use the cyanine dyes of the present invention as compounds for recording layers, have a satisfactory recording/reading property, they are high in PRSNR (Partial Response SNR) when conducted in accordance with the HD DVD-R standard, Ver. 1.0, defined by DVD Forum. Concretely, the optical recording media attain a PRSNR level of at least 25, preferably, at least 30, and more preferably, at least 40. Optimum recording power should preferably be low and the optical recording media will attain their maximum PRSNR levels by a laser beam power of not greater than 10 mW, preferably, not greater than 9 mW, and more preferably, not greater than 8 mW. PRSNR is an index that can express S/N (signal-to-noise) of reading signals and simultaneously express both the actually reproduced wave form and the theoretical linearity of PR wave form, and it is an index required for estimating bit error

rate of disc.

The optical recording media, which use the cyanine dyes of the present invention as compounds for recording layers, should never be restricted to have a specific construction, however, they are usually constructed by combining substrates, recording layers, reflection layers, protection layers, and printing layers (layers for label), where conventional constructions and production methods are applicable.

The substrates usable in the present invention can be conventional ones and are usually processed with appropriate materials, for example, processed into discs, 12 cm in diameter and 0.6 mm or 1.1 mm in thickness, to meet final use by the methods such as compression molding, injection molding, compression-injection molding, photopolymerization method (2P method), thermosetting integral method, and lightsetting integral method. Such discs can be used in a single plate or used after appropriately attaching each other with adhesives or adhesive sheets, etc. In principal, any materials for substrates can be used in the present invention as long as they are substantially transparent and have a transmittance of at least 80%, and preferably at least 90% over the wavelengths ranging from 400 to 500 nm. Examples of such materials are glasses, ceramics, and others such as plastics such as poly(methyl methacrylate), polycarbonate, polystyrene (styrene copolymer), polymethylpenten, polyetherimide, polyethersulfone, polyarylate, polycarbonate/polystyrene alloy, polyestercarbonate, polyphthalateaarbonate, polycarbonateacrylate, non-crystalline polyolefin, methacrylate copolymer, diallylcarbonatediethylene glycol, and epoxy resin, among which polycarbonate is frequently used.

The preferred embodiments according to the present invention are explained with reference to the following Examples.

Example 1

<Cyanine dye>

An adequate amount of acetic anhydride was placed in a reaction vessel, admixed with 1.8 g of the compound represented by Chemical Formula 31 and 2.6 g of the compound represented by Chemical Formula 32, and the mixture was refluxed for one hour while heating and then cooled after dropping water thereunto. The formed crystal was collected and recrystallized with methanol to obtain 0.8 g of a yellow crystal of the compound represented by Chemical Formula 33.

Chemical Formula 31:

[Chem. 39]

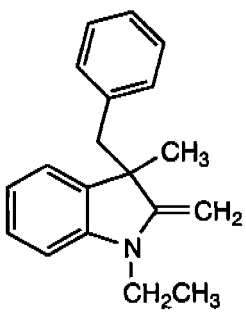

Chemical Formula 32:

[Chem. 40]

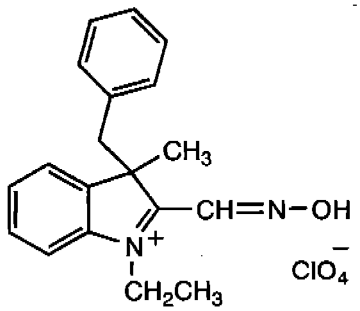

Chemical Formula 33:

[Chem. 41]

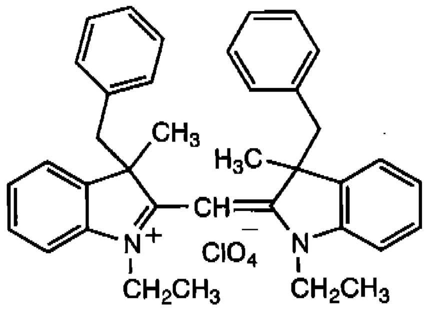

Thereafter, an adequate amount of acetonitrile was placed in a reaction vessel, admixed with 0.7 g of the previously obtained compound represented by Chemical Formula 33 and 0.9 g of the azo metal complex represented by Chemical Formula 34, followed by dissolving the compounds under heating and stirring conditions and then removing the acetonitrile to obtain 0.9 g of a brown colored crystal of the cyanine dye represented by Chemical Formula 1 of the present invention.

Chemical Formula 34:

[Chem. 42]

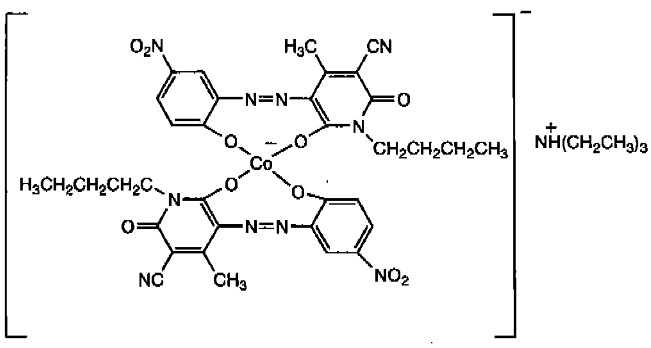

A part of the crystal was sampled and measured for its melting- and decomposition-points on DSC analysis, revealing that the cyanine dye of this Example showed a melting point and a decomposition point at around 190°C and 250°C, respectively. The melting point means a peak top temperature of endothermic peak during temperature increase on DSC analysis, while the decomposition point means an onset temperature of exothermic peak induced by decomposition during temperature increase on DSC analysis. These are applicable to the following measurements. As light absorption property, conventional measurement for the visible absorption spectrum of the cyanine dye of this Example

in methanol solution revealed that it has a main absorption maximum (ε=8.62 x $10^4$) at a wavelength of around 469 nm in the violet to the blue regions. Conventional measurement for the solubility of the cyanine dye of this Example in organic solvents at 20°C revealed that it has satisfactory solubilities with no actual hindrance in organic solvents of amides such as N,N-dimethylformamlde, methanol, TFP, methylethylketone, acetonitrile, and chloroform; and of alcohols, ketones, nitriles, and halogens. Upon mass spectrometric analysis, the cationic and anionic parts of the cyanine dye of this Example showed parent ion peaks of $(M-H)^+$ at 511.31 (m/e) and $(M-H)^-$ at 797.15 (m/e), respectively. While, the compound represented by Chemical Formula 33 showed a decomposition point at around 212°C and an absorption maximum (ε=3.60 x $10^4$) at a wavelength of 447 nm in methanol solution.

Since the cyanine dye of this Example efficiently absorbs short-wavelength visible light and has satisfactory dissolvability and heat characteristics, it can be advantageously used as a light-absorbing material for shielding the visible light and for utilizing energy of the visible light by absorbing short-wavalength visible light in various fields of, for example, information recordings, solar energy generations, electric machinery apparatuses, electric communicating apparatuses, optical apparatuses, clothes, building/bedding/decorating products, sanitary and health goods, and agricultural materials.

Example 2

<Cyanine dye>

Except for replacing the compounds represented by Chemical Formulae 31 and 32 with the compounds represented by Chemical Formulae 35 and 36, the reaction materials were similarly reacted as in Example 1 to obtain a yellow and orange colored crystal of the compound represented by Chemical Formula 37. Similarly as in Example 1, the compound represented by Chemical Formula 37 and the azo metal complex represented by Chemical Formula 34 were reacted to obtain a brown colored crystal of the cyanine dye of the present invention represented by Chemical Formula 20.

Chemical Formula 35:

[Chem. 43]

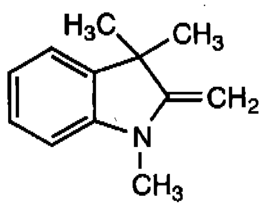

Chemical Formula 36:

[Chem. 44]

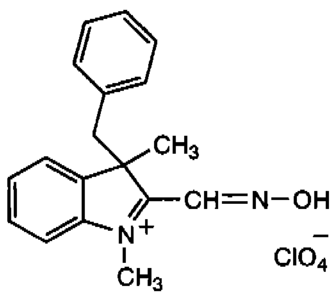

Chemical Formula 37:

[Chem. 45]

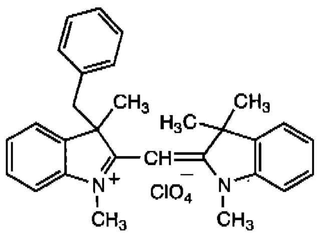

A part of the crystal was sampled and measured for its melting- and decomposition-points, as indexes ofheat characteristics, on DSC analysis, revealing that the cyanine dye of this Example showed a melting point and a decomposition point at around 270°C and 273°C, respectively. As light absorption property, conventional measurement for the visible absorption spectrum of the cyanine dye of this Example in methanol solution revealed that it has a main absorption maximum at a wavelength of around 454 nm in the violet to the blue regions ($\varepsilon$=7.39 x $10^4$). Conventional measurement for the solubility of the cyanine dye of this Example in organic solvents at 20°C revealed that it has satisfactory solubilities with no actual hindrance in organic solvents of amides, alcohols, ketones, nitriles, and halogens such as N,N-dimethylformamide, methanol, TFP, methylethylketone, acetonitrile, and chloroform. Upon mass spectrometric analysis, the cationic and anionic parts of the cyanine dye of this Example showed parent ion peaks of $(M-H)^+$ at 407.25 (m/e) and $(M-H)^-$ at 797.15 (m/e), respectively. Upon $^1$H-nuclear magnetic resonance analysis (abbreviated as "$^1$H-NMR analysis", hereinafter), the cyanine dye of this Example in a chloroform deuteride solution showed a chemical shift $\delta$ (ppm, TMS) at peaks of 0.50 (6H, t, $CH_3$-), 0.75 to 0.88 (4H, m, -$CH_2$-), 0.94 to 1.10 (4H, m, -$CH_2$-), 1.64 (3H, s, $CH_3$-), 1.65 (3H, s, $CH_3$-), 1.76 (3H, s, $CH_3$-), 2.93 (6H, s, $CH_3$-), 2.93 (3H, br, $CH_3$-), 3.07 (3H, br, $CH_3$-), 3.20 (1H, d, Ar-CH-), 3.47 (1H, d, Ar-CH-), 3.59 to 3.65 (4H, m, -$CH_2$-), 5.44 (1H, s, -CH=), 6.67 (2H, d, ArH), 6.82 (2H, d, ArH), 7.04 to 7.15 (4H, m, ArH), 7.40 to 7.51 (7H, m, ArH), 7.99 (2H, dd, ArH), and 9.13 (2H, d, ArH). While, the compound represented by Chemical Formula 37 showed a decomposition point at around 238°C and an absorption maximum at a wavelength of 439 nm in methanol solution ($\varepsilon$=3.64 x $10^4$).

Since the cyanine dye of this Example efficiently absorbs short-wavelength visible light and has satisfactory dissolvability and heat characteristics, it can be advantageously used as a light-absorbing material for shielding the visible light and for utilizing energy of the visible light by absorbing short-wavelength visible light in various fields of, for example, information recordings, solar energy generations, electric machinery apparatuses, electric communicating apparatuses, optical apparatuses, clothes, building/bedding/decorating products, sanitary and health goods, and agricultural materials.

Example 3

<Cyanine dye>

Except for replacing the compounds represented by Chemical Formulae 31 and 32 with the compounds represented by Chemical Formulae 38 and 39, the reaction materials were similarly reacted as in Example 1 to obtain a yellow colored crystal of the compound represented by Chemical Formula 40. Similarly as in Example 1, the compound represented by Chemical Formula 40 and the azo metal complex represented by Chemical Formula 34 were reacted to obtain a red and orange colored crystal of the cyanine dye of the present invention represented by Chemical Formula 22.

Chemical Formula 38:

[Chem. 46]

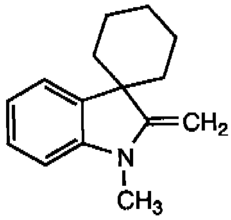

Chemical Formula 39:

[Chem. 47]

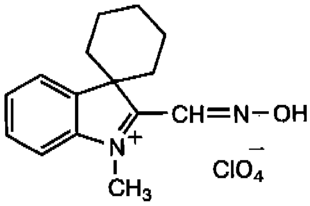

Chemical Formula 40:

[Chem. 48]

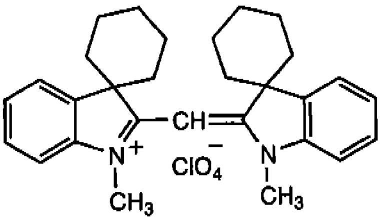

A part of the crystal was sampled and measured for its melting-and decomposition-points, as indexes of heat characteristics, on DSC analysis, revealing that the cyanine dye of this Example showed a melting point and a decomposition point at around 270°C and 273°C, respectively. As light absorption property, conventional measurement for the visible absorption spectrum of the cyanine dye of this Example in methanol solution revealed that it has a main absorption maximum at a wavelength of around 454 nm in the violet to the blue regions ($\varepsilon$=7.39 x $10^4$). Conventional measurement for the solubility of the cyanine dye of this Example in organic solvents at 20°C revealed that it has satisfactory solubilities with no actual hindrance in organic solvents of amides, alcohols, ketones, nitriles, and halogens such as N,N-dimethylformamide, methanol, TFP, methylethylketone, acetonitrile, and chloroform. Upon $^1$H-NMR analysis, the cyanine dye of this Example in a chloroform deuteride solution showed a chemical shift $\delta$ (ppm. TMS) at peaks of 0.50 (6H, t, $CH_3$-), 0.75 to 0.90 (4H, m, -$CH_2$-), 0.95 to 1.05 (4H, m, -$CH_2$-), 1.50 to 2.10 (20H, m, $CH_3$-), 2.93 (6H, s, $CH_3$-), 3.25 (6H, s, $CH_3$-). 3.55 to 3.70 (4H, m, -$CH_2$-), 5.48 (1H, s, -CH=), 6.83 (2H, d, ArH), 7.34 to 7.40 (4H, m, ArH), 7.52 (2H, t. ArH), 7.87 (2H, d, ArH), 7.99 (2H, dd, ArH), and 9.13 (2H, d, ArH). While, the compound represented by Chemical Formula 40 showed a decomposition point at around 227°C and an absorption maximum at a wavelength of 434 nm in methanol solution ($\varepsilon$=3.62 x $10^4$).

Since the cyanine dye of this Example efficiently absorbs short-wavelength visible light and has satisfactory dissolvability and heat characteristics, it can be advantageously used as a light-absorbing material for shielding the visible light and for utilizing energy of the visible light by absorbing short-wavelength visible light in various fields of, for example, information recordings, solar energy generations, electric machinery apparatuses, electric communicating

apparatuses, optical apparatuses, clothes, building/bedding/decorating products, sanitary and health goods, and agricultural materials.

Example 4

<Cyanine dye>

Except for replacing the compounds represented by Chemical Formulae 31 and 32 with the compounds represented by Chemical Formulae 35 and 39, the reaction materials were similarly reacted as in Example 1 to obtain a yellow and orange colored crystal of the compound represented by Chemical Formula 41. Similarly as in Example 1, the compound represented by Chemical Formula 41 and the azo metal complex represented by Chemical Formula 34 were reacted to obtain a red and orange colored crystal of the cyanine dye of the present invention represented by Chemical Formula 21.

Chemical Formula 41:

[Chem. 49]

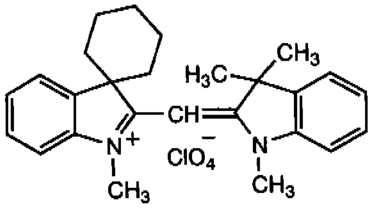

A part of the crystal was sampled and measured on DSC analysis for its melting- and decomposition-points, as indexes of heat characteristics, revealing that the cyanine dye of this Example showed a melting point and a decomposition point at around 270°C and 273°C, respectively. As light absorption property, conventional measurement for the visible absorption spectrum of the cyanine dye of this Example in methanol solution revealed that it has a main absorption maximum at a wavelength of around 454 nm in the violet to the blue regions ($\varepsilon$=7.39 x $10^4$). Conventional measurement for the solubility of the cyanine dye of this Example in organic solvents at 20°C revealed that it has satisfactory solubilities with no actual hindrance in organic solvents of amides, alcohols, ketones, nitriles, and halogens such as N,N-dimethylformamide, methanol, TFP, methylethylketone, acetonitrile, and chloroform. Upon $^1$H-nuclear magnetic resonance ($^1$H-NMR) analysis, the cyanine dye of this Example in a chloroform deuteride solution showed a chemical shift $\delta$ (ppm. TMS) at peaks of 0.50 (6H, t, $CH_3$-), 0.75 to 0.90 (4H, m, -$CH_2$-), 0.95 to 1.05 (4H, m, -$CH_2$-), 1.64 (6H, s, $CH_2$-), 1.50 to 2.10 (10H. m, -$CH_2$-), 2.94 (6H, s, $CH_3$-), 3.27 (3H, s, $CH_3$-), 3.36 (3H, s, $CH_3$-), 3.55 to 3.70 (4H, m, -$CH_2$-), 5.44 (1H, s, -CH=), 6.83 (2H, d, ArH), 7.31 to 7.56 (7H, m, ArH), 7.88 (1H, d, ArH), 8.00 (2H, dd, ArH), and 9.13 (2H, d, ArH). While, the compound represented by Chemical Formula 41 showed a decomposition point at around 240°C and an absorption maximum at a wavelength of 435 nm in methanol solution ($\varepsilon$-3.59 x $10^4$).

Since the cyanine dye of this Example efficiently absorbs short-wavelength visible light and has satisfactory dissolvability and heat characteristics, it can be advantageously used as a light-absorbing material for shielding the visible light and for utilizing the energy of the visible light by absorbing short-wavelength visible light in various fields of, for example, information recordings, solar energy generations, electric machinery apparatuses, electric communicating apparatuses, optical apparatuses, clothes, building/bedding/decorating products, sanitary and health goods, and agricultural materials.

Varying somewhat the production conditions and yields depending on the structures of the cyanine dyes of the present invention, all of the cyanine dyes, which include those represented by Chemical Formulae 1 to 30 other than the above-identified cyanine dyes, can be obtained in a desired amount by the methods in Examples 1 to 4 or in accordance therewith.

Example 5

<Lightfastness of cyanine dyes>

Fifteen milligrams of anyone of the cyanine dyes represented by Chemical Formulae 1 and 20 to 22 obtained by the methods in Examples 1 to 4 was weighed and added to three milliliters of TFP and dissolved therein by energization

with ultrasonics at ambient temperature for five minutes. The solution was in conventional manner dropped to homogeneity over the surface of either side of a polished glass substrate. 5 cm x 5 cm, followed by rotating the glass substrate at a rate of 1,000 rpm/min for one minute to homogeneously apply the solution over the substrate and then drying the substrate by blowing hot air or cold air in this order to form a thin membrane of any one of the cyanine dyes of the present invention.

The cyanine dyes were measured for transmittance ($T_0$) at the wavelengths of their absorption maxima (about 450 nm), while a 7.5 W xenon lamp was fixed apart from the glass substrate at a prescribed intervals and irradiated therewith (irradiated energy of 180 $W/m^2$ when measured on the surface of the substrate) for 5.5 hours while blowing cold air to the substrate. Thereafter, the substrates were immediately measured for transmittance (T) at the wavelengths of absorption maxima of the cyanine dyes and the residual percentages (%) thereof were calculated by assigning the obtained transmittances T and $T_0$ to the equation 1. In parallel, similarly as in the above, thin membranes, as controls 1 to 4, formed with any one of the compounds represented by Chemical Formulae 33, 37, 40 and 41; thin membranes, as controls 5 to 8, formed with any one of the compounds represented by Chemical Formulae 33, 37, 40 and 41 in combination with the azo metal complex presented by Chemical Formula 34 as a light resistant improver; and thin membranes, as controls 9 to 10, formed with any one of the compounds represented by Chemical Formulae 42 and 43 , and these thin membranes were similarly treated as in the above, and measured for transmittances T and $T_0$ at wavelengths of their absorption maxima for use as controls. The results are in Table 1.

[Equation 1]

$$\text{Residual percentage (\%) of dye} = \frac{100 - T}{100 - T_0} \times 100$$

[Table 1]

| Cyanine dye | Lightfastness improver | Residual percentage (%) of dye | Remarks |
|---|---|---|---|
| Chemical Formula 1 | Non | 99.3 | Present invention |
| Chemical Formula 20 | Non | 99.1 | Present invention |
| Chemical Formula 21 | Non | 99.6 | Present invention |
| Chemical Formula 22 | Non | 99.8 | Present invention |
| Chemical Formula 33 | Non | 29.4 | Control 1 |
| Chemical Formula 37 | Non | 31.2 | Control 2 |
| Chemical Formula 40 | Non | 35.6 | Control 3 |
| Chemical Formula 41 | Non | 33.8 | Control 4 |
| Chemical Formula 33 | Chemical Formula 34 | 89.9 | Control 5 |
| Chemical Formula 37 | Chemical Formula 34 | 90.3 | Control 6 |
| Chemical Formula 40 | Chemical Formula 34 | 91.5 | Control 7 |
| Chemical Formula 41 | Chemical Formula 34 | 93.8 | Control 8 |
| Chemical Formula 42 | Non | 15.3 | Control 9 |
| Chemical Formula 43 | Non | 92.8 | Control 10 |

Chemical Formula 42:

[Chem. 50]

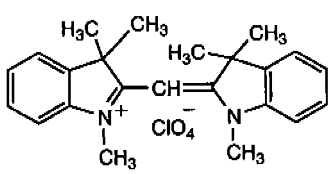

Chemical Formula 43:

[Chem. 51]

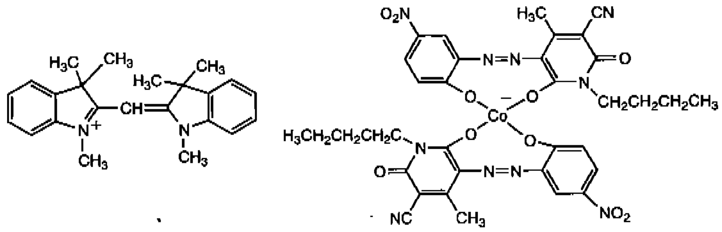

As evident from the results in Table 1, in the thin membranes as controls 1 to 4, which consisted of any one of Chemical Formulae 33, 37, 40 and 41, these compounds had changed in considerable amounts with only a 5.5 hour exposure, i.e., their initial absorbances lowered to give residual percentages (%) of 29.4 to 35.6% of their initial levels. Although not so distinct as found in the thin membranes as controls 1 to 4, the thin membranes, which consisted of the azo metal complex represented by Chemical Formula 34 and any one of the compounds represented by Chemical Formulae 33, 37, 40 and 41, gave lowered residual percentages (%) of 89.9 to 93.8% of their initial levels by the similar exposure as in the above. While the thin membranes, which were constructed by any one of the cyanine dyes of the present invention represented by Chemical Formulae 1 and 20 to 22, did not substantially lower in their absorption abilities as they had gave residual percentages (%) of 99% or higher even when exposed to light similarly as in the above. The thin membranes of controls 9 and 10, which were constructed by related compounds represented by Chemical Formulae 42 and 43, gave lowered residual percentages (%) of 15.3% and 92.8% when exposed to light similarly as in the above.

These experimental results show that the cyanine dyes, which have a specific structure composed of monomethine cyanine dyes as cations and specific metal complexes, particularly, azo metal complexes as anions, are outstandingly superior in lightfastness in the visible region to compounds composed of, as counter ions, anions other than the above-identified specific metal complexes, or mere mixtures of the above-identified compounds and metal complexes.

Example 6

A solution, prepared by mixing the dye represented by Chemical Formula 20 with TFP as a solvent to give a concentration of 1.0% by weight, was applied by spin coating over the surface of a polycarbonate substrate, 0.6 mm in thickness, having trucks with a track pitch of 0.4 μm and a groove width of 60 nm. The substrate thus obtained had an absorbance of 0.32 at a wavelength of 470 nm, where the air was measured as a reference. Thereafter, the substrate was dried at 70°C for 25 min, installed with an AgBi0.2Nd0.5 reflection membrane, 120 nm in thickness, by spattering, and adhered to a back substrate, 0.6 mm in thickness, using an ultraviolet-ray hardening resin to prepare an optical recording medium.

Using a tester commercialized by Pulstec Industries Co., Inc, with a laser beam wavelength of 405 nm and NA of 0.65, the optical recording medium was recorded at a line velocity of 6.61 m/s and a minimum mark length of 204 nm. Recording and reading were conducted in accordance with the HD DVD-R standard, Ver. 1.0, specified by DVD Forum and evaluated based on PRSNR (Partial Response SNR) specified in the above standard.

As a result, the optical recording medium showed a highly satisfactory result; it gave an optimum recording power of 7.0 mW and a PRSNR level of 40.0 far exceeding the standard level of 15, as shown in FIG. 1. The optical recording medium was revealed to have "LOW-TO-HIGH" recording mechanism and have a satisfactory recording property because it gave a great difference in reflection rates of 7.4% and 18.0%, corresponding to those before and after recording, respectively.

Example 7

Except for replacing the dye used in Example 6 with the one represented by Chemical Formula 22, another optical recording medium was prepared similarly as in Example 6 and evaluated by the similar method as in Example 6.

As a result, the optical recording medium showed a satisfactory result; it gave an optimum recording power of 8.4 mW and a PRSNR level of 31.9 far exceeding the standard level of 15, as shown in FIG. 1. The optical recording medium was revealed to have "LOW-TO-HIGH" recording mechanism and have a satisfactory recording property because it gave a great difference in reflection rates of 11.9% and 23.3%, corresponding to those before and after recording information, respectively.

Example 8

<Optical recording medium>

Except for replacing the dye used in Example 6 with any one of the dyes represented by Chemical Formulae 1 to 19, 21 and 23 to 30, optical recording media, which have satisfactory recording properties of the same or higher optimum recording power, PRSNR, and reflection rates as those of the one in Example 7, can be prepared under the similar conditions as in Example 6.

Industrial Applicability

As described above, since the cyanine dyes of the present invention have improved lightfastness, efficiently absorb short-wavelength visible light, exhibit satisfactory solubility in solvents without any actual hindrance, and have improved heat characteristics, they can be quite advantageously used as light absorbing materials for shielding and utilizing short-wavelength visible light by absorbing the light in various fields of, for example, information recordings, solar energy generations, electric machinery apparatuses, electric communicating apparatuses, optical apparatuses, clothes, building/bedding/decorating products, sanitary and health goods, and agricultural materials. Particularly, they can be quite advantageously useful as compounds for recording layers in optical recording media which record information by using blu-ray semiconductor lasers.

As described hitherto, the present invention with such outstanding functions and effects is a significant invention that greatly contributes to this art.

**Claims**

1. A cyanine dye represented by General Formula 1:

   General Formula 1:

   [Chem. 53]

   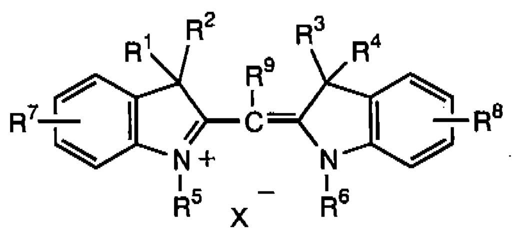

   (wherein in General Formula 1, $R^1$ through $R^4$ are the same or different hydrocarbon groups, and at least one of which either binds to a cyclic group that may optionally have a substituent or form a cyclic structure by binding together with other hydrocarbon group that binds to the same carbon atom. $R^5$ and $R^6$ are the same or different hydrocarbon groups that may have substituents. $R^7$ through $R^9$ represent hydrogen atoms or appropriate substituents. $X^-$ represents an anion of metal complex having, as a central atom, a transition element from the 5 to 12 groups in the periodic law table.)

2. The optical recording medium of claim 1, wherein said $X^-$ in General Formula 1 is an anion of azo metal complex.

3. An optical recording medium, which comprises the cyanine dye of claim 1 or 2.

4. The optical recording medium of claim 3, which records and reads information by using a light with a wavelength of not shorter than 300 nm but not longer than 500 nm.

[F I G. 1]

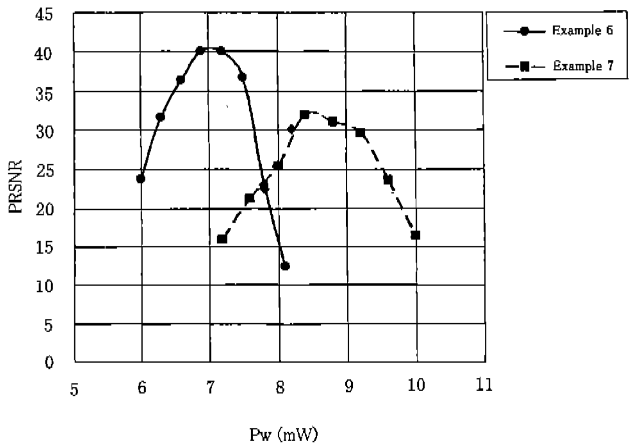
45
40
35
30
25
20
15
10
5
0
PRSNR
5
6
7
8
9
10
11
Pw (mW)
Example 6
Example 7

**INTERNATIONAL SEARCH REPORT**

International application No.
PCT/JP2006/310051

A. CLASSIFICATION OF SUBJECT MATTER
***C09B69/02***(2006.01), ***C09B23/00***(2006.01), ***C09B45/14***(2006.01), ***C09B45/18***(2006.01), ***C09B45/20***(2006.01), ***C09B45/22***(2006.01), ***C07D209/14***(2006.01), ***C07D209/96***(2006.01), ***C07D213/84***(2006.01), ***C07D239/60***(2006.01),
According to International Patent Classification (IPC) or to both national classification and IPC

B. FIELDS SEARCHED

Minimum documentation searched (classification system followed by classification symbols)
B41M5/26, C07D209/14, C07D209/96, C07D213/84, C07D239/60, C07D401/12, C07D405/14, C07D409/14, C09B23/00, C09B45/14, C09B45/18, C09B45/20, C09B45/22, C09B69/02, G11B7/244

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | WO 02/50210 A1 (Kabushiki Kaisha Hayashibara Seibutsu Kagaku Kenkyusho), 27 June, 2002 (27.06.02), & EP 1347030 A1 & US 2003/64322 A1 | 1-4 |
| A | WO 98/29257 A1 (TDK Corp.), 09 July, 1998 (09.07.98), & EP 887202 A1 & US 6168843 B1 | 1-4 |
| P,A | WO 2005/092988 A1 (Kabushiki Kaisha Hayashibara Seibutsu Kagaku Kenkyusho), 06 October, 2005 (06.10.05), (Family: none) | 1-4 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>12 July, 2006 (12.07.06) | Date of mailing of the international search report<br>25 July, 2006 (25.07.06) |
| Name and mailing address of the ISA/<br>Japanese Patent Office<br>Facsimile No. | Authorized officer<br>Telephone No. |

Form PCT/ISA/210 (second sheet) (April 2005)

**INTERNATIONAL SEARCH REPORT**

International application No.
PCT/JP2006/310051

C (Continuation). DOCUMENTS CONSIDERED TO BE RELEVANT

| Category* | Citation of document, with indication, where appropriate, of the relevant passages | Relevant to claim No. |
|---|---|---|
| P,A | WO 2005/083011 A1 (Kabushiki Kaisha Hayashibara Seibutsu Kagaku Kenkyusho), 09 September, 2005 (09.09.05), (Family: none) | 1-4 |

Form PCT/ISA/210 (continuation of second sheet) (April 2005)

**INTERNATIONAL SEARCH REPORT**

International application No.
PCT/JP2006/310051

Continuation of A. CLASSIFICATION OF SUBJECT MATTER
(International Patent Classification (IPC))

***C07D401/12***(2006.01), ***C07D405/14***(2006.01), ***C07D409/14***(2006.01), ***B41M5/26***
(2006.01), ***G11B7/244***(2006.01)

(According to International Patent Classification (IPC) or to both national classification and IPC)

Form PCT/ISA/210 (extra sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 1116611 A
- JP 2002020592 A
- JP 2003167343 A
- JP 2002074740 A
- WO 00075111 A


### Non-patent literature cited in the description


- KANKO-SHIKISO. Sangyo Tosho Publisher, 17 October 1997, 24-30
- Shikizai-Kogaku-Handbook. Asakura Publishing Co., Ltd, 25 November 1989, 1, 274-1282
- Senryo-to-Yakuhin. 1992, vol. 37, 185-197